# EUROPEAN PATENT SPECIFICATION

(11) **EP 0 395 427 B1**
(45) Date of publication and mention of the grant of the patent: **08.06.1994**
(21) Application number: 90304592.0
(22) Date of filing: 27.04.1990
(51) Int. Cl.: A61K 6/00, C08F 30/02, A61K 6/083

(54) **Command-curable composition**
Zusammensetzung mit beherschbarer Vernetzung
Composition à réticulation sur commande

(30) Priority: 27.04.1989 GB 8909614
(43) Date of publication of application: 31.10.1990
(73) Proprietor: BRITISH TECHNOLOGY GROUP LIMITED, London SE1 6BU (GB)
(72) Inventor: Ellis, John, East Molesey, Surrey KT8 0HY (GB); Wilson, Alan Donald, Liphook, Hampshire GU30 7HH (GB)
(74) Representative: Neville, Peter Warwick

(56) References cited:
- EP-A- 0 035 162
- EP-A- 0 219 058
- EP-A- 0 241 277
- EP-A- 0 340 016
- PATENT ABSTRACTS OF JAPAN vol. 8, no. 241 (C-250)(1678) November 6, 1984
- & JP-A-59 122 410 (TOKYAMA SODA K.K. )
- PATENT ABSTRACTS OF JAPAN vol. 8, no. 11 (C-205)(1448) January 18, 1984& JP-A-58 177 907 (TOKYAMA SODA K.K. )

## Description

This invention relates to compositions curable on command, e.g. by the action of incident energy e.g. light (ultraviolet or visible) or ultrasound or a chemical initiator. Such compositions may contain inert or reactive fillers, in which case they can be classed as cements, and may be particularly useful in surgical, especially dental, applications.

As dental cements, glass ionomers as described in for example GB Patents 1422337 and 1484454 have attained wide popularity for their compressive strength, their inherent adhesion to tooth material, their relatively fast setting time and their anti-caries action. However, a drawback in clinical practice is that, once mixed, the glass ionomer cement composition stays workable for a strictly limited time only, and sets rapidly.

EP-A-0 219 058 discloses a dental cement composition comprising polymerisable unsaturated monomers including acid, salt etc. groups, fillers, e.g. glass which can react with the acid, salt etc. groups, and a hardener, e.g. benzoyl peroxide. The monomer may be a compound with at least two phosphonate groups or two polymerisable groups such as 1-methacryloxyethane-1,1-diphosphonic acid.

According to the present invention, a command-curable composition comprises (i) a phosphonate ester which is, or is chemically identical to, the reaction product between a plurality of unsaturated phosphonic acid molecules and a polyhydric alcohol molecule in the mole ratio (0.2 - 2.0) phosphonic acid groups: 1 hydroxyl groups, (ii) an initiator, and (iii) a cross-linkable polymeric acid capable of being catalysed by any multivalent cation, said acid (iii) containing on average one phosphonic acid group per one to three backbone carbon atoms. The initiator is suitably a light-activated initiator system or may be a chemical initiator system. The said mole ratio is preferably (0.5 - 1.5):1 such as (0.8 - 1.2):1. The hydroxyls in the polyhydric alcohol are preferably interconnected via from two to twenty such as two to six carbon atoms, with oxygen atoms optionally interposed at (preferably) a frequency of at least one per five carbon atoms; the polyhydric alcohol is preferably a diol e.g. bis-(2-hydroxyethyl) ether (1,5-dihydroxy-3-oxa-pentane). (i) may be di(vinyl phosphonic acid) ester. (iii) may be poly(vinyl phosphonic acid).

Optionally the composition additionally comprises any one or more of cation-leachable (e.g. aluminosilicate) glass powder, amphoteric or basic metal oxide (e.g. MgO), and water.
A minor proportion of poly(carboxylic acid) such as poly(acrylic acid) may also be present.

In the case of a chemically activated initiator system, its components (unlike the light-initiated case) must be kept separate until the composition is to be cured.

The invention extends to a pack comprising two separated pastes which when mixed form a curable composition as set forth above; the first paste may be the acid(s) plus water and the light-activated initiator system, and the second paste may be the glass powder suspended in the phosphonate ester. If the two pastes have been formulated to appropriate concentrations, one could in use squeeze out equal lengths of paste from two tubes, or scoop out equal numbers of spoonfuls from two tubs, as an easy way to ensure that the mixture is of the correct composition.

The glass powder preferably consists of particles substantially all of which are smaller than 100 microns, preferably smaller than 60 microns. The Si:Al range of 0.6 - 2:1 yields an opaque product, which may be acceptable in appropriate cases, but 0.2 - 0.6:1 can also be used. In place of glass, MgO deactivated at at least 900°C may be used.

The invention also extends to a pack comprising the components of the curable composition set forth above, so packed that when unpacked and mixed they form the composition.

The invention will now be described by way of example.

Various esters of vinyl phosphonic acid 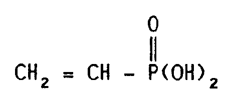
with polyhydric alcohols were formed, using the reaction of a precursor of this acid (vinyl phosphonic dichloride) with the alcohol in the presence of water, thus: 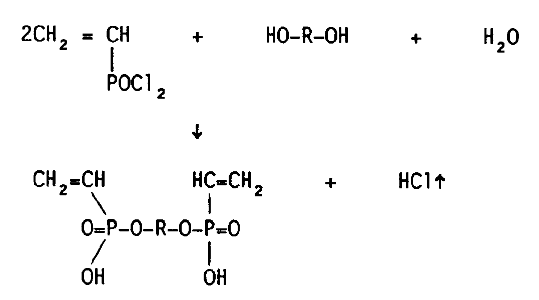
R = -C₂₋₆- with optional intervening oxygens e.g. -C₂H₄-O-C₂H₄- or -C₂H₄- or -nC₄H₈- or 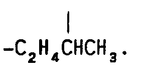
We term this reaction product a divinylphosphonic acid ester (DVPA ester).

Preferably, an intervening oxygen is provided after at most five consecutive carbon atoms. Thus, R(OH)₂ may (in addition) be butane 1,3 diol, pentane 1,5 diol, neopentylglycol, or H(OCH₂CH₂)ₙOH having a molecular weight of 200-600, i.e. n = 7 to 20 (approximately).

The DVPA ester can be induced to polymerise by the action of suitable initiator systems, a polymerisation taking place through the vinyl groups, like the dimethacrylate resins currently used in other dental restorative materials. Since the DVPA ester has two vinyl groups, crosslinking will also occur. The acid groups contained by DVPA ester confer water solubility on it.

These DVPA esters were mixed with visible-light-activated initiators (camphorquinone CQ, plus ethyl dimethylaminobenzoate EDMAB, plus sodium p-toluenesulphinate NaTS) and exposed to visible light. They cured within 60 seconds to hard, water resistant materials. The presence of water in the DVPA ester did not prevent setting, although a high proportion of water resulted in softer materials initially, but which set over a longer period (about 1 hour). Similarly the presence of glass powder in the DVPA ester/water mix had no effect on the curing of the materials.

In the foregoing reaction, it will be appreciated that the ratio of (dibasic) vinylmonophosphonic acid molecules CH₂=CH-PO(OH)₂ or CH₂=CH-POCl₂ molecules to dihydric alcohol molecules can be varied; where this ratio is >1:1 i.e. the degree of esterification is correspondingly less than 100%, some acid function will remain on the DVPA ester. When, as in the reaction scheme above, the ratio is 2:1, the degree of esterification will be 50% and the other 50% of the acid OH groups remain. We can express this more generally in terms of the ratio between phosphonic acid groups -PO(OH)₂ and alcohol -OH groups; when this ratio is 1:1 the degree of esterification will be 50%. DVPA ester in the presence of suitable initiators and activators will form a crosslinked polymer still containing some acid groups. In addition, in the presence of an ion-leachable glass and a poly(vinyl phosphonic acid), acid-base reactions will also occur.

The presence of a water-soluble oxygen-containing unsaturated phosphonated monomer (i.e. the ester) has the useful additional effect of slowing down the acid-base (PVPA-glass) reaction, which is normally too fast unless other measures are taken, e.g. heat-deactivation of the glass.

### Example 1

Vinyl phosphonyl chloride was reacted with half the number of moles of bis-(2-hydroxyethyl)ether to form DVPA ester

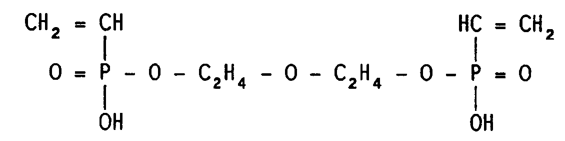

This DVPA ester was mixed with CQ + EDMAB + NaTS (as light-activated initiators), to form a composition according to the invention, and exposed to 60 seconds' white light. The resulting cement was hard and resistant to water.

In an identical example except that the composition also included a minor proportion of water, similar results were obtained.

### Example 2

A composition was made up consisting of 4 parts by weight of the ester of Example 1, water (1 part), an aluminosilicate glass powder (8 parts) and CQ + EDMAB + NaTS. The glass is prepared by mixing together 437 parts by weight silica, 230 parts by weight alumina, 129 parts by weight calcium fluoride, 175 parts by weight cryolite and 29 parts by weight aluminium phosphate and heating to 1300°C for 75 minutes. The melt is cooled rapidly by pouring into water. The resulting glass is ground and sieved, and the fraction of particle size less than 45 microns used in the composition. It was exposed to 60 seconds' white light, and the resulting cement was hard, opaque and resistant to water.

### Example 3

A composition was made up consisting of
0.25g of the DVPA ester of Example 1
0.02ml water
0.04g poly(vinyl phosphonic acid)
0.5g of the glass of Example 2
0.01g of light-activated initiator (consisting of CQ+EDMAB+NaTS).

The whole was thoroughly mixed to form a paste, in which the poly(vinyl phosphonic acid) solid was dissolved in a mixture of the water and the ester.

One portion of this composition remained workable for about 30 minutes in normal indoors light. Another portion, exposed to 60 seconds' white light, was immediately stable against water. A further composition (not according to the invention) was made up according to this Example but without the initiators; that composition hardened overnight to a water-stable cement.

### Example 4

A two paste composition was devised comprising firstly a concentrated solution (80% by mass and hence adequately viscous) of poly(vinyl phosphonic acid) in water, additionally containing CQ + EDMAB + NaTS; and secondly a paste containing the phosphonate ester of Example 1 (0.5g) and the glass powder (1.0g) of Example 2. Equal volumes of the two pastes were mixed and on exposure to white light the mixture set to a hard, water stable cement.

## Claims

1. A command-curable composition, comprising (i) a phosphonate ester which is, or is chemically identical to, the reaction product between a plurality of unsaturated phosphonic acid molecules and a polyhydric alcohol molecule in the mole ratio (0.2 - 2.0) phosphonic acid groups:l hydroxyl groups, (ii) an initiator, and (iii) a cross-linkable polymeric acid capable of being catalysed by any multivalent cation, said acid (iii) containing on average one phosphonic acid group per one to three backbone carbon atoms.

2. A composition according to Claim 1, wherein said mole ratio is (from 0.5 to 1.5):1.

3. A composition according to Claim 1 or 2, wherein the hydroxyls in the polyhydric alcohol are interconnected via from two to twenty carbon atoms.

4. A composition according to Claim 3, wherein the hydroxyls in the polyhydric alcohol are interconnected via from two to six carbon atoms.

5. A composition according to Claim 3 or 4, wherein oxygen atoms are interposed in the alcohol.

6. A composition according to Claim 5, wherein an oxygen atom is interposed at a frequency of at least one per five carbon atoms.

7. A composition according to any preceding claim, wherein the ester (i) is or comprises di(vinyl phosphonic acid) ester.

8. A composition according to any preceding claim, wherein the polymeric acid (iii) is or comprises poly(vinyl phosphonic acid).

9. A composition according to any preceding claim, further comprising any one or more of cation-leachable glass powder, amphoteric or basic metal oxide, and water.

10. A composition according to Claim 9, wherein the glass powder is of particles substantially all smaller than 100 microns.

## Revendications

1. Composition durcissable sur commande, comprenant (i) un ester phosphonate qui est le produit de réaction entre une multiplicité de molécules d'acide phosphonique insaturé et une molécule de polyol dans le rapport molaire (0,2-2,0) groupes d'acide phosphonique pour un groupe hydroxyle, ou qui lui est chimiquement identique, (ii) un initiateur, et (iii) un acide polymère réticulable susceptible d'être catalysé par tout cation multivalent, cet acide (iii) contenant en moyenne un groupe acide phosphonique pour 1 à 3 atomes de carbone du squelette.

2. Composition selon la revendication 1, dans laquelle ledit rapport molaire est (0,5 à 1,5):1.

3. Composition selon la revendication 1 ou la revendication 2, dans laquelle les groupes hydroxyle dans le polyol sont reliés entre eux par l'intermédiaire de 2 à 20 atomes de carbone.

4. Composition selon la revendication 3, dans laquelle les groupes hydroxyle dans le polyol sont reliés entre eux par l'intermédiaire de 2 à 6 atomes de carbone.

5. Composition selon la revendication 3 ou la revendication 4, dans laquelle des atomes d'oxygène sont interposés dans l'alcool.

6. Composition selon la revendication 5, dans laquelle un atome d'oxygène est interposé à une fréquence d'au moins 1 pour 5 atomes de carbone.

7. Composition selon l'une des revendications précédentes, dans laquelle l'ester (i) est ou contient un ester d'acide divinyl phosphonique.

8. Composition selon l'une des revendications précédentes, dans laquelle l'acide polymère (iii) est ou contient un acide polyvinyl phosphonique.

9. Composition selon l'une des revendications précédentes, contenant en outre toute poudre de verre contenant un ou plusieurs cations lessivables, un oxyde métallique amphotère ou basique et de l'eau.

10. Composition selon la revendication 9, dans laquelle pratiquement toutes les particules de la poudre de verre sont plus petites que 100 »m.

## Patentansprüche

1. Auf Befehl härtbare Masse, umfassend (i) einen Phosphonatester, der das Reaktionsprodukt zwischen einer Anzahl von ungesättigten Phosphonsäure-Molekülen und einem mehrwertigen Alkoholmolekül im Molverhältnis (0,2 - 2,0) Phosphonsäuregruppen zu einer Hydroxylgruppe ist oder damit chemisch identisch ist, (ii) einen Initiator und (iii) eine polymere vernetzbare Säure, die katalysiert werden kann durch ein mehrwertiges Kation, wobei die Säure (iii) im Mittel eine Phosphonsäuregruppe auf ein bis drei Hauptketten-Kohlenstoffatome enthält.

2. Masse nach Anspruch 1, wobei das Molverhältnis (0,5 bis 1,5):1 ist.

3. Masse nach Anspruch 1 oder 2, wobei die Hydroxylgruppen in dem mehrwertigen Alkohol über zwei bis zwanzig Kohlenstoffatome miteinander verbunden sind.

4. Masse nach Anspruch 3, wobei die Hydroxylgruppen in dem mehrwertigen Alkohol über zwei bis sechs Kohlenstoffatome miteinander verbunden sind.

5. Masse nach Anspruch 3 oder 4, wobei Sauerstoffatome in den Alkohol (Hauptkette) eingebaut sind.

6. Masse nach Anspruch 5, wobei mindestens ein Sauerstoffatom pro fünf Kohlenstoffatome eingebaut ist.

7. Masse nach einem der vorangehenden Ansprüche, wobei der Ester (i) Di(vinylphosphonsäure)ester ist oder umfaßt.

8. Masse nach einem der vorangehenden Ansprüche, wobei die polymere Säure (iii) Poly(vinylphosphonsäure) ist oder umfaßt.

9. Masse nach einem der vorangehenden Ansprüche, umfassend ferner eine oder mehrere der folgenden Substanzen, durch Glaspulver mit auslaugbaren Kationen, amphotere oder basische Metalloxide und Wasser.

10. Masse nach Anspruch 9, wobei das Glaspulver aus Teilchen besteht, die im wesentlichen alle kleiner kleiner als 100 »m sind.
